# EUROPÄISCHE PATENTSCHRIFT

(11) **EP 1 330 440 B1**
(45) Veröffentlichungstag und Bekanntmachung des Hinweises auf die Patenterteilung: **21.11.2007**
(21) Anmeldenummer: 01951478.5
(22) Anmeldetag: 04.05.2001
(51) Int. Cl.: C07D 219/10, C07D 487/04, A01N 1/02

(54) **PATHOGENINAKTIVIERENDES MITTEL MIT EINEM AN NUKLEINSÄUREN BINDENDEN ELEMENT UND DESSEN VERWENDUNG**
AGENT WHICH INACTIVATES PATHOGENS, COMPRISING AN ELEMENT THAT BONDS WITH NUCLEIC ACIDS AND THE USE THEREOF
AGENT INACTIVANT DES AGENTS PATHOGENES, COMPRENANT UN ELEMENT QUI SE LIE AUX ACIDES NUCLEIQUES, ET SON UTILISATION

(30) Priorität: 18.10.2000 DE 10051628
(43) Veröffentlichungstag der Anmeldung: 30.07.2003
(73) Patentinhaber: Fresenius HemoCare Beteiligungs GmbH, 60323 Frankfurt am Main (DE)
(72) Erfinder: NEUMANN, Hans-Jürgen, 66606 St. Wendel (DE); KNOLLER, Helmut, 89073 Ulm (DE)
(74) Vertreter: Laufhütte, Dieter
(86) Internationale Anmeldenummer: PCT/EP2001/005034
(87) Internationale Veröffentlichungsnummer: WO 2002/032875

(56) Entgegenhaltungen:
- EP-A- 0 720 851
- WO-A-94/20090
- WO-A-96/39818
- WO-A-98/30545

## Beschreibung

Die Erfindung betrifft ein Mittel zur Inaktivierung von pathogenen Keimen wie Viren und Bakterien und dessen Verwendung vorzugsweise in physiologischen Lösungen wie Blut oder Blutbestandteilen. Insbesondere besteht das pathogeninaktivierende Mittel aus einem an Nucleinsäuren bindenden Element und einem die Wirkung der Nukleinsäure zerstörenden Konjungat sowie optional einem dazwischen befindlichen Spacer, wobei das Konjungat ein Metallchelatkomplex ist.

Mit zunehmender Ausbreitung pathogener Keime wird es immer schwieriger, geeignete Mittel zur Inaktivierung der Keime zu finden. Die Problematik wird dadurch verschärft, daß gerade Viren eine hohe Variabilität aufweisen, die früher eingesetzte antivirale Mittel wirkungslos machen. Dabei sollte es möglich sein, ein pathogeninaktivierendes Mittel zum einem sehr spezifisch zu gestalten, um eine Virenart gezielt abzutöten, zum anderen aber auch soweit unspezifisch zu gestalten, daß eine Großzahl pathogener Keime inaktiviert wird.

Dazu bietet sich an, wie dies auch in weiten Bereichen des Standes der Technik bereits geschehen ist, als Angriffsziel des pathogenen Keimes seine Nukleinesäuren zu wählen, also die DNA (desoxiribonucleic acid) oder RNA (ribonucleic acid) des pathogenen Keimes. Im Falle einer spezifischen Anlagerung des pathogeninaktivierenden Mittels kann man eine sehr selektive Inhibierung erreichen wie dies in Nucleic Acid Research, Vol. 15 Nr. 23(1), Seite 9909 bis 9919 die Autoren Zerial et al. zeigen, indem sie die selektive Inhibierung von Typ A Influenza Viren mittlels eines hochspezifischen Oligonukleotids mit einem reaktiven Konjungat beschreiben. Diese Selektivität ist darauf zurückzuführen, daß das einzusetzende Oligonukleotid spezifisch als Komplementärstrang zu einer konservativen DNA-Sequzenz synthetisiert wurde. Dabei paaren bekanntlich die Basen Cytosin stets mit Guanin und Thymin bzw. sein Gegenstück in der RNA, Uracil stets mit Adenin. Diese Adhäsionskräfte sind zum einem temperaturabhängig, zum anderen abhängig von der Menge der zueinander passenden Paare. Mit dieser Methode läßt sich demnach spezifisch eine Virensorte oder eine Klasse von Viren mit Gemeinsamkeiten inhibieren.

In der WO 95/32986 und in der WO 92/05284 sind antisense Oligonukleotide beschrieben, welche die pathogenen Keime inhibierenden Eigenschaften aufweisen. Dabei besteht das Oligonukleotid jeweils aus Sequenzen, die ebenfalls zu der Nukleinsäuresequenz des pathogenen Keimes komplementär sind oder im Falle der WO 92/05284 an spezifische strategische Seiten der Mikroben binden. Somit werden nur spezifische pathogene Zielkeime erfasst und die Oligonukleotide sind demnach nicht für verschiedenen pathogenen Keime, insbesondere nicht für alle Virenarten geeignet.

Um auch Viren abtöten zu können, werden oft Stoffe verwendet, die eine hohe oxidierende oder alkylierende Wirkung haben und/oder sich an das Genom anlagern. Daher sind diese Stoffe auch für andere Zellen mutagen oder cancerogen. Weiterhin stellt sich das Problem, daß sie dennoch für eine große Zahl von Viren aufgrund deren Heterogenität versagen. Um auch Viren zu aktivieren, sollte eine Anlagerung an Nukleinsäuren zwar erfolgen, aber möglichst unspezifisch.

Im Falle einer unspezifischen Anlagerung wird gibt es 4 Gruppen von Anlagerungsreagenzien, die auch in der US 5,691,132 beschrieben sind, die Interkalatoren, die sog. "groove binders", wobei diese in "minor groove binder" und "major groove binder" unterteilt sind. Dabei sind Interkalatoren Stoffe, die sich zwischen die beiden Nukleinsäurestränge der DNA legen. Eine DNA-Doppelhelix weist größere Rillen in der Spirale und kleinere auf, in die sich die "major -" bzw. "minor groove binder" einlagern können. Weiterhin sind die Stoffe, die an das Phosphatgruppengerüst von Nukleinsäuren binden sowie unspezifische Oligonukleotide, wie sie auch ausführlich in der prioritätsbegründenen DE 100 51 628.9 genannt sind.

Insbesondere bei der Verwendung von Blutprodukten beispielsweise bei der Transfusion von humanem Blut oder bei der Gewinnung von Proteinen aus Blut oder Blutbestandteilen besteht eine großer Bedarf, potentiell pathogene Keime abzutöten, bevor die Produkte ihrer bestimmungsmäßigen Verwendung zugeführt werden. Im Stand der Technik sind bereits diverse Methoden und Stoffe bekannt, die eine Inaktivierung von pathogenen Keimen bewirken. Allen gemeinsam sind entweder belastende Nebenwirkungen oder eine aufwendige Handhabung.

In der US 5,691,132 wird ein Verfahren beansprucht, Pathogene in einem Blutprodukt zu inaktivieren, wobei ein Interkalator zu dem Blutprodukt gegeben wird, der durch seine unspezifische Anlagerung an die DNA der Pathogene bereits eine Zerstörung der DNA bewirken kann. Allerdings ist dieser Interkalator in der Lage, auch andere DNA anzugreifen und wirkt dadurch mutagen bzw. cancerogen. Daher muß er nach erfolgter Inaktivierung mittels eines Adsorbens wieder aus dem Blutprodukt entfernt werden, wodurch ein weiterer Handlungsschritt erforderlich ist, der zudem nicht ungefährlich für den Durchführenden ist.

Auch die WO 00/04930 zeigt ein Verfahren und eine Vorrichtung zur Inaktivierung von Mikroorganismen auf, wobei die Anlagerung an die Nukleinsäure der Mikroorganismen mittels eines nicht toxischen Stoffes erfolgen soll, der mittels Bestrahlung erst aktiviert wird und die DNA zerstört. Derartige Stoffe sind Alloxazine bzw. Isoalloxazine, zu denen auch Riboflavin oder Vitamin B2 gehört. Obgleich Riboflavin seiner benzoiden Mehrringstruktur wegen zu den Interkalatoren gehören sollte, scheint er keine mutagenen oder cancerogenen Eigenschaften aufzuweisen. Erst bei Lichteinstrahlung weist Riboflavin die pathogeninaktivierende Wirkung. Nachteilig an dieser Methode ist die Bestrahlung, die ein zusätzlicher Bearbeitungsschritt darstellt, wie auch schädigend auf andere Zellen wirken kann. Des weiteren entstehen unerwünschte Nebenprodukte, beispielsweise kann Riboflavin zu Lumiflavin oder Lumichrome umgewandelt werden.

Weiterhin ist bekannt, Strangbrüche in der DNA oder RNA zu induzieren durch ein Konjungat mit einer reaktiven Gruppe, wie beispielsweise ein EDTA-Eisen Chelatkomplex (EDTA=EthylenDiaminTetraAcetat). In der Literaturstelle Gene 72 (1988), Seite 361-371 zeigen die Autoren Boidet-Foget et al., daß ein spezifisches Schneiden von einzelsträngiger und doppelsträngiger DNA möglich ist. Auch ist in Biochemistry Vol. 29 Nr. 6 (Feb. 13, 1990) von Celander und Chech gezeigt worden, daß das Eisen (II)-EDTA katalysierte Schneiden von RNA und DNA keine oder nur geringe Spezifität hat.

Diese Eigenschaft ist in der Molekularbiologie demnach seit Jahren bekannt und wird für ein sogenanntes "mapping" genutzt. Dabei bindet meist ein Protein an eine spezifische Stelle der DNA, wobei diese Stelle, die durch das Protein geschützt sind, durch die Fe(II)-Komplexe nicht angegriffen werden kann. Dadurch ist es möglich, Anbindungsstellen an einem Gen zu identifizieren. Beispiele für ein derartiges mapping finden sich in Biochemistry (1994), Vol. 33, Seite 9831 bis 9844 oder Biochemistry Vol. 35 Nr. 37 (Sept. 7, 1996) Seite 11931ff.

Aufgabe der vorliegenden Erfindung ist es, ein Mittel zur Inaktivierung von Viren bereitzustellen, welches die Nachteile der im Stand der Technik bekannt gewordenen Pathogen inaktivierenden Mittel, beispielsweise eine aufwendige Handhabung nicht aufweist. Die Aufgabe wird durch die Merkmale des ersten Anspruchs gelöst.

Kennzeichnend für die Erfindung ist die funktionelle Trennung des pathogeninaktivierenden Mittels in zwei Bereiche, wobei ein Bereich für die Anbindung an Nukleinsäuren zuständig ist, und der zweite Bereich für die Zerstörung der Nukleinsäuren. Dabei kann die Anbindung spezifisch oder unspezifisch erfolgen, ist jedoch vorzugsweise unspezifisch, da möglichst alle pathogenen Keime erreicht werden sollten.

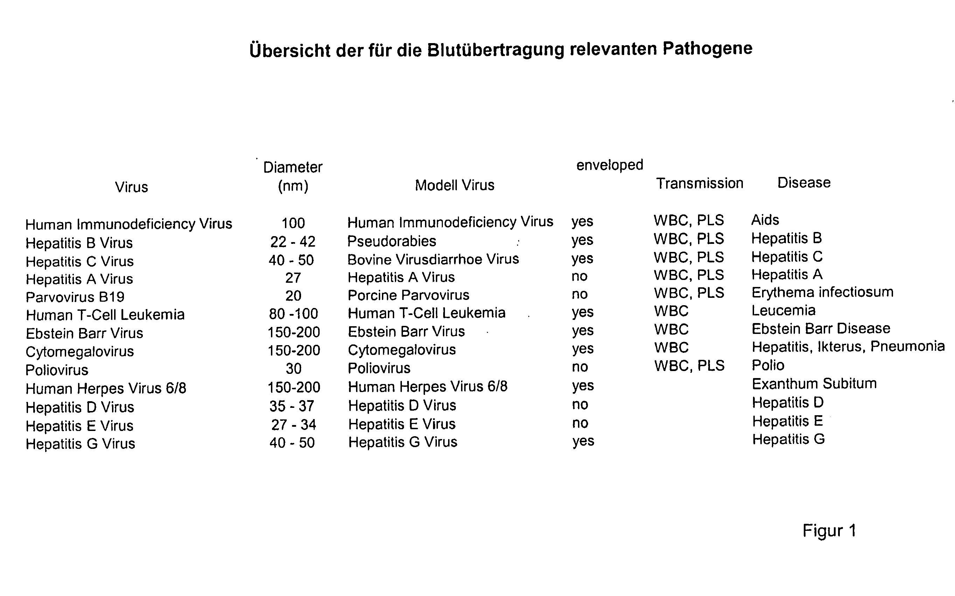

Dabei wird unter pathogenen Keimen alle für Organismen zu Krankheitsbildern führenden Keime mit einem Genom verstanden, wie Bakterien oder Viren. In Figur 1 sind tabellarisch die wichtigsten bei einer Blutübertragung relevanten Viren und Bakterien aufgezeigt. Daraus ist zu ersehen, daß manche Viren doppelsträngige DNA, andere einzelsträngige RNA besitzen, Viren behüllt oder unbehüllt sein können.

Während Interkalatoren, Phophatbrücken-Binder oder "groove-binder" per se unspezifisch an DNA binden, gibt es bei Oligonukleotiden die Möglichkeit, durch komplementäre Basenpaarbildung die gewünschte Spezifität einzustellen. Dabei wird die Spezifität umso geringer, je kleiner das Oligonukleotid ist. Eine geringe Spezifität wird durch die Länge eines Oligonukleotides von maximal 6 spezifischen Nukleotiden erreicht, wobei spezifisches Nukleotid bedeutet, daß ihre Base aus der Gruppe der spezifischen Basen Guanin, Adenin, Thymin, Uracil und/oder Cytosin ausgewählt ist. Statistisch findet ein derartiges synthetisches Oligonukleotid mehrere Treffer auf einem Genom, so daß möglichst alle pathogenen Keime, vor allem möglichst alle Virenarten inaktiviert werden können. Wird die Nukleotidfolge aus spezifischen Nukleotiden länger als 6 Nukleotide, werden die Treffer auf dem Genom erheblich kleiner, wodurch die Spezifität und als Folge davon auch die Selektivität steigt.

Außer den 5 oben genannten spezifischen Basen existieren auch sogenannte universellen Basen, die mit allen oder zumindest mehreren Basen eine Basenpaarbildung eingehen. Diese können - wie das 5-Nitroindol - synthetisch sein, oder wie das Inosin natürlich vorkommend. Inosin, welches eine Base der Transfer RNA (tRNA) ist, kann mit Cytosin, Adenosin und Uracil eine Basenpaarbildung eingehen. Bei einer Verwendung des Inaktivierungsmittels in Blut oder Blutprodukten, die für eine Transfusion vorgesehen sind, ist dementsprechend Inosin als universelle Base bevorzugt. Aber auch andere universelle Basen sind für die Verwendung in vitro denkbar.

Der Einsatz einer universellen Base ermöglicht eine Bindung des Oligonukleotides bei höheren Temperaturen, wobei aber der Vorteil erhalten bleibt, möglichst viele Bindungen mit Genomen pathogener Keime zu erhalten mit nur wenigen spezifischen Nukleotiden. Dabei entscheidet auch die Länge des Genoms über die Häufigkeit einer Anlagerung.

Ein Oligonukleotid ist auch fähig, an eine doppelsträngige DNA (dsDNA) zu binden und mit ihr eine sogenannte Triple-Helix zu bilden. Es hat sich gezeigt, daß eine Folge gleicher Nukleotiden mit den Basen Thymin oder Cytosin besonders vorteilhaft an dsDNA binden. Aus diesem Grund wird bevorzugt eine derartige Struktur verwendet, besonders bevorzugt eine Folge von mindestens drei Cytosinen, da Cytosin eine etwa doppelte Bindungsstärke an DNA aufweist als Thymin.

Des weiteren wird vorteilhafterweise eine Seite des Oligonukleotides mit einer Schutzgruppe versehen, die den Abbau des Oligonukleotides zumindest verzögert. Für eine in vivo Anwednung wird auch hierfür wieder ein weiteres Nukleotid verwendet, da dieses natürlich vorkommt und im menschlichen und tierischen Körper nicht giftig wirken kann. Um ihre Wirksamkeit als Schutzgruppe entfalten zu können, wird aber das weitere Nukleotid spiegelsymmetrisch zu den anderen Nukleotiden eingebaut, so daß möglichst wenig den RNAsen oder DNAsen, also den nukleotidabbauende Enzymen, eine Angriffsmöglichkeit geboten wird. Dieses Schutzgruppen-Nukleotid trägt nicht zur Bindung an einen Komplementärstrang bei und wird daher bei der Längenbestimmung eines Oligonukleotides nicht mitgezählt.

Beispiele für Stoffe, die an das Phosphatgruppengerüst binden, sind Spermine, Spermidine und andere Polyamine zu nennen, "major groove binders" sind beispielsweise die Aflatoxine und zu den "minor groove binders" zählen unter anderem die Triarylmethan-Farbstoffe oder Hoechst 33258- und andere Hoechst Farbstoffe, Berenil, DAPI (4', 6'-Diamidin-2-Phenylindol), Distamycin, Mitomycin, Netropsin und andere Lexitropsine.

Die größte Gruppe der an DNA bindenden Substanzen stellen jedoch die Interkalatoren. Diese sind in der Regel flache aromatische Systeme, die durch ihr π-Elektronensystem eine Brücke zwischen den Doppelsträngen statt der sonst vorhandenen Wasserstoffbrücken bilden. Dabei können diese Aromaten heterozyklisch sein oder benzoid, meist jedoch sind Interkalatoren aus Mehrringsystemen zusammengesetzt.

Als Vertreter der Gruppe sind zu nennen: Acridine, Acridone, Proflavin, Acriflavine, Aloxazine, Isoalloxazine, Porphine bzw. Porphyrine, Actinomycin, Anthracyclinon, beta-Rhodomycin A, Daunamycin, Thiaxanthenone, Miracil D, Anthramycin, Mitomycin, Echinomycin, Quinomycin, Triostin, Diacridine, Ellipticene (auch Dimere, Trimere und Analoga), Norphilin A, Fluorene und Fluorenone, Fluorenodiamine, Quinacrin, Benzacridine, Phenazine, Phenanthradine, Phenothiazine, Chlorpromazine, Phenoxazine, Benzothiazole, Xanthene und Thioxanthene, Anthraquinone, Anthrapyrazole, Benzothiopyranoindole, 3,4-Benzpyrene, Benzopyrendiolepoxid, 1-Phenyloxirane, Benzanthracen-5,6-oxid, Benzodipyrone, Quinolone, Chloroquine, Quinine, Phenylquinolinecarboxamid, Furocoumarine, wie Psoralen und Isopsoralen, Ethidiumsalze, Propidium und Coralyne, Ellipticin-Katione und deren Derivate, polycyclische Hydrocarbone und ihre Oxiranderivate und Echinomycin.

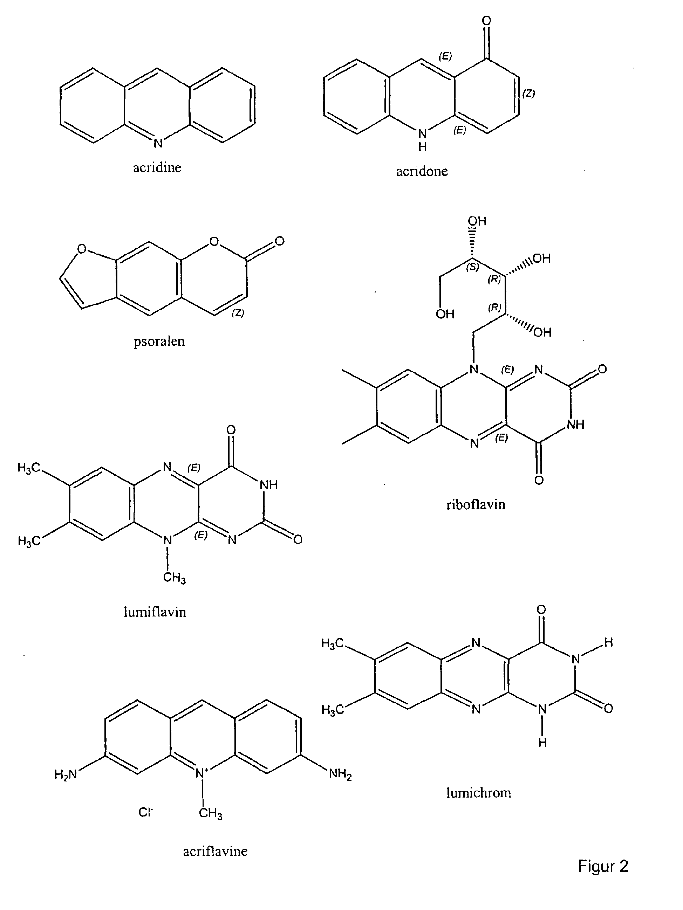

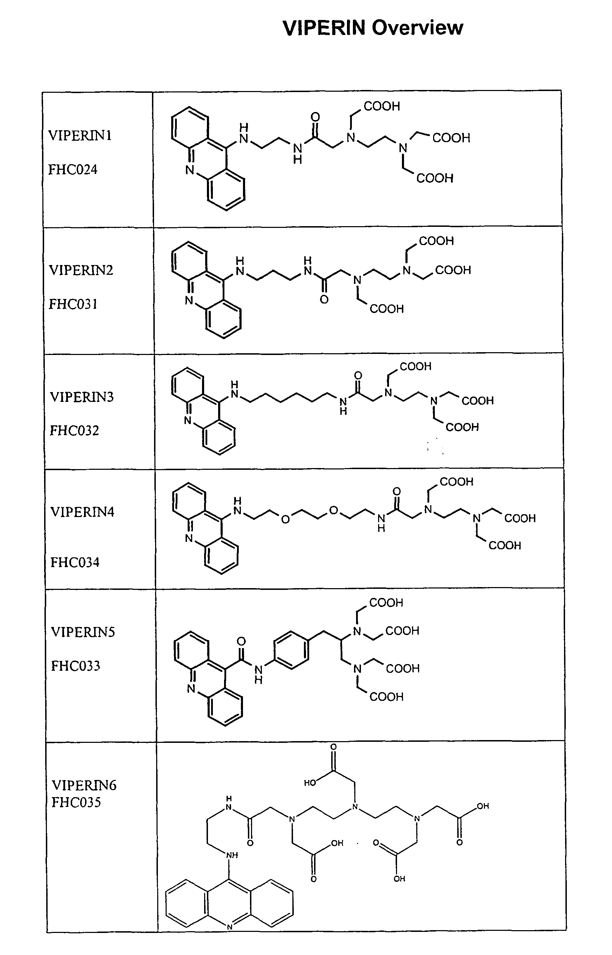

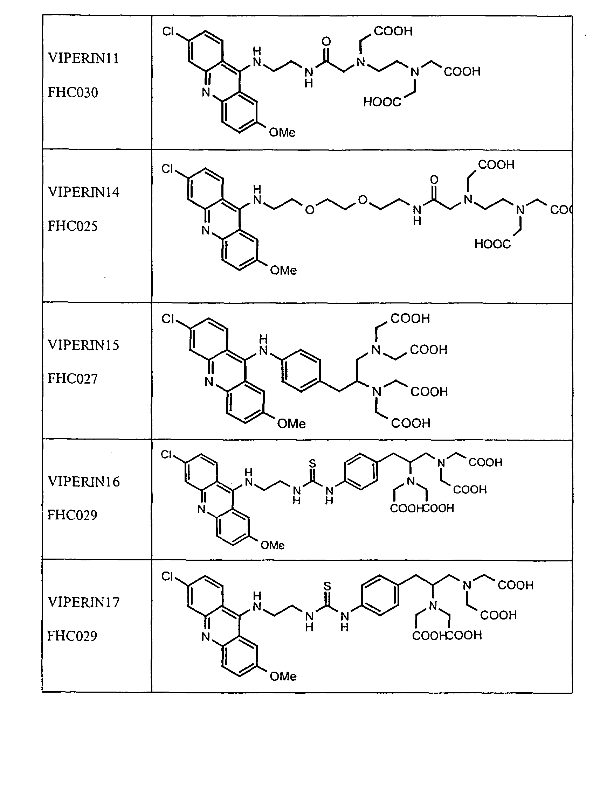

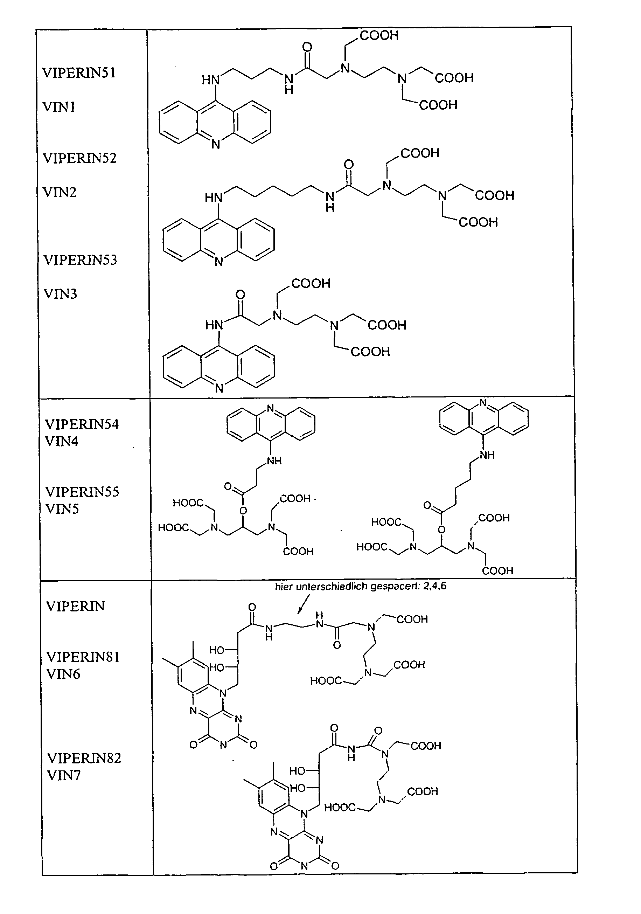

Bevorzugt sind erfindungsgemäß Acridine, ein dreifaches 6-Ring-System und deren Derivate, wie Alloxazine und Isoalloxazine sowie Porphine bzw. substituierte Porphine, die Porphyrine, und deren Derivate. Diese Stoffe sind zum einen wegen ihrer starken Bindungsfähigkeit ausgewählt zum anderen wegen der Tatsache, daß für eine Anwendung in biologischen Systemen aus dieser Gruppe auch nicht toxische, nicht mutagen und nicht cancerogen wirkende Derivate ausgewählt werden können. So besteht beispielsweise Häm, ein Bestandteil des Blutes aus einem mit Fe(II) besetzten, aus 4 Indolen hergestellten Porphyrinring. Ebenso ist unter den Alloxazinen das Vitamin B2 oder auch Riboflavin zu finden, welches als das nukleinsäurebindende Element besonders bevorzugt ist. In Figur 2 sind geeignete Mehrringsystem dargestellt.

Die eigentliche Zerstörung des Genoms erfolgt durch das an das freie Ende des Oligonukleotides angehängte reaktive Konjungat, welches ein Metali-Chelatkomplex ist, wobei das Metall zwischen zwei Oxidationsstufen wechseln kann. Die pathogeninhibierende Wirkung wird wahrscheinlich durch Schneiden der Nukleinsäuren gemäß dem Fenton Mechanismus ausgelöst und geht dabei demnach von der reduzierenden Eigenschaft des Metalls aus. Demnach wird das Metall selbst oxidiert und kann durch den Einsatz eines Reduktionsmittels wieder regeneriert werden.

Erfindungsgemäß können alle Komplexe, die eine Zerstörung des Genoms der Pathogene bewirken, eingesetzt werden. Komplexe bestehen aus einem Zentralatom oder erfindungsgemäß einem Zentralion und Liganden. Als Zentralionen fungieren meist Schwermetallkationen hoher Ladung mit kleinen Ionenradien. Häufig sind die Liganden Anionen, aber auch ungeladene Moleküle treten als Liganden auf. Handelt es sich um Verbindungen, die zwei oder mehrere funktionelle Gruppen besitzen, also mehrere Koordinationsstellen des Zentralions besetzen können, spricht man von bi- oder multidentalen Liganden, wie Ethylendiamin oder Oxalat. Die Ladung des Komplexes entspricht der Summe der Ladung der ihn bildenden Einzelionen.

Die Anwesenheit von zwei oder mehr Koordinationsstellen in einem organischen Molekül führt zur Ringbildung. Sie erfolgt vorzugsweise, wenn sich ein spannungsfreier 5- oder 6-Ring bilden kann. Allgemein werden Verbindungen, in denen ein Molekül über ein Metallion durch Koordination zu einem Ring geschlossen ist, als Chelatkomplexe oder Chelate bezeichnet. Als innere Chelatkomplexe werden ungeladene Chelatkomplexe bezeichnet, die Ladungen des Zentralions und die der ringbildenden Liganden kompensieren sich, wie im wie Nickel-Diacethyldioxim oder Magnesium-Oxinat, um beispielhaft bekannte Komplexe zu nennen.

Die sogenannten Komplexone, deren wichtigster Vertreter EDTA und die Nitrilotriessigsäure darstellen, sind Komplexbildner, die mit fast allen Kationen, einschließlich der Erdalkaliionen, zum Teil sehr stabile, wasserlösliche Komplexe bilden. Als weiteres Beispiel sei das Eisen EDPA (Diethylenaminpentaacetat) genannt.

Erfindungsgemäß bevorzugt ist ein Eisen(II)-EDTA-Chelatkomplex bzw. ein Eisen(III)-EDTA-Chelatkomplex. Insbesonders im Einsatz in biologischen Systemen wie zur Inaktivierung von Viren in Blut oder Blutbestandteilen ist dieser Komplex besonders geeignet, da sowohl Eisen als auch EDTA biokompatibel sind sowie bereits zugelassene Medikamente zur Förderung des menschlichen Blutbildes und daher bei Infusion in den menschlichen oder tierischen Körper bedenkenlos eingesetzt werden können. Besonders vorteilhaft ist für eine derartige Verwendung die Kombination mit ebenfalls einem biokompatiblen Element, wie beispielsweise das genannte Riboflavin. Bei Verwendung eines Spacers zwischen dem Eisen-EDTA-Komplex und Riboflavin bietet sich demnach ein ebenfalls biokompatibler Spacer oder Linker an.

Dabei kann das Eisen in den Oxidationsstufen 2+ oder 3+ vorliegen. Liegt das Eisen in der Oxidationsstufe 2+ vor, so kann jedes Molekül Eisen einmal seine Wirkung entfalten und wird dabei selbst zu Eisen (III) oxidiert. Indem das Eisen (III) nun wiederum durch den Einsatz eines weiteren Reduktionsmittels wieder zu Eisen (II) reduziert wird, ist die Wirkung des Komplexes beliebig oft wiederholbar, so daß nur geringe Mengen des pathogeninaktivierenden Mittels eingesetzt werden müssen. Auch als Reduktionsmittel wird man in vivo ein unbedenklicher, biokompatibler Stoff verwenden, beispielsweise Vitamin C oder Ascorbinsäure, respektive ihr Salz Ascorbat. Des weiteren ist der Einsatz von Fe(III) grundsätzlich denkbar, so daß die Inaktivierungsreaktion durch die erfindungsgemäße Zugabe des Reduktionsmittels erst starten kann.

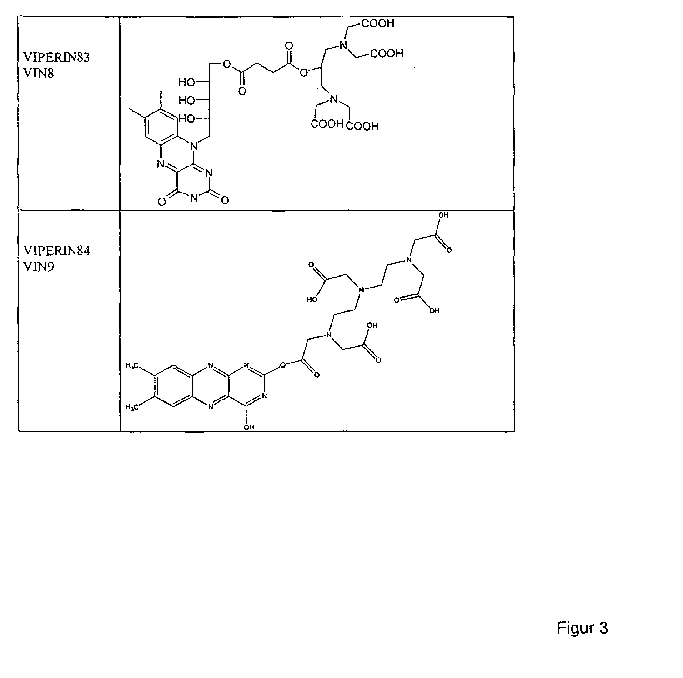

Die erfindungsgemäße Kombination von einem an Nukleinsäure bindenden Element, gegebenenfalls einem Spacer und dem Nukleinsäure zerstörenden Konjungat wird fortan als VIPERIN bezeichnet. Figur 3 zeigt verschiedene VIPERINE, wobei VIPERIN81 oder VIN6 die oben genannte bevorzugte Ausführung aus einer Verbindung von Riboflavin und Eisen-EDTA, aufzeigt. Mit VIPERIN 1 sind alle nachfolgend aufgezeigten Versuche ausgeführt worden.

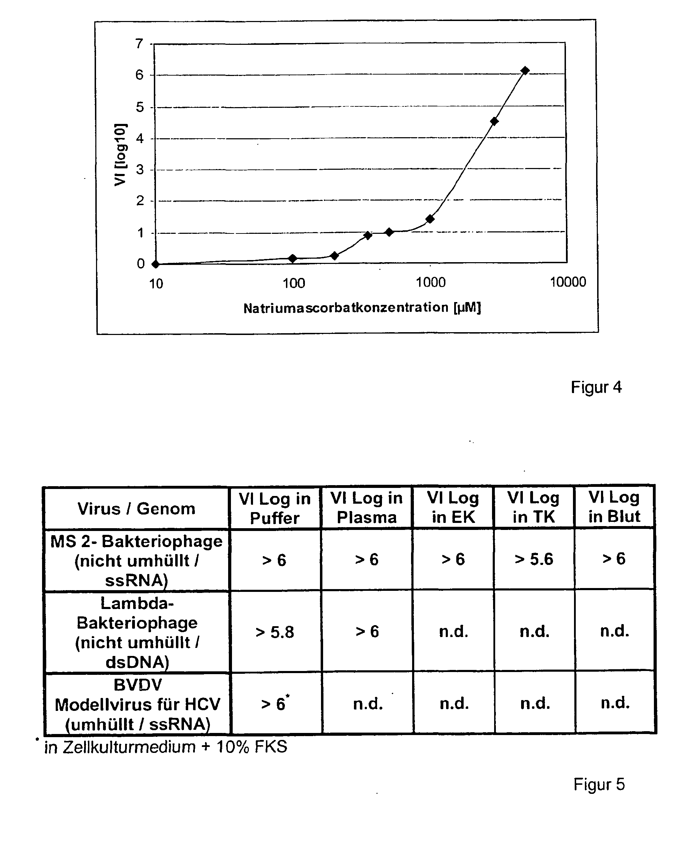

Die oben erwähnte Virusaktivierung ist in Figur 4 in Abhängigkeit von der Natriumascorbatkonzentration dargestellt:

Ein Phagentiter eines MS2-Phagen von 1x10⁹ pfu/ml Wasser wurde 4 Stunden bei Raumtemperatur mit 50µg/ml VIPERIN1 inkubiert. Der Acridingrundkörper eignet sich dazu, an die Nukleinsäuren des Phagen MS2 zu binden, während der angehängte Eisen-EDTA-Komplex die Zerstörung der Nukleinsäure initiiert. Dabei ist VIPERIN1 mit Eisen(III) beladen (nicht gezeigt in Figur 3). Ab einer Natriumascorbat-Konzentration von 100µmol/l steigt die Vireninaktivierung (VI), aufgetragen im 10-er Logarithmus stetig an. Je höher die Ascorbatkonzentation, desto mehr Eisen(III) wird zu Eisen(II) reduziert, der "aktiven" Form des EDTA-Eisen-Komplexes und desto aktiver ist demnach auch das Nukleinsäure zerstörende Konjungat. Interessanterweise ist die Konzentration von Ascorbat im menschlichen Körper etwa 100µM, so daß davon auszugehen ist, daß in Blut oder Blutbestandteilen keine oder nur geringe Aktivierung des Eisen(III)-EDTA Komplexes erfolgt.

Zur Untermauerung dieser These wurde der MS2-Bakteriophage als nicht umhüllter Virus mit einzelsträngiger RNA (ssRNA) verschiedenen Medien, auch physiologischen Medien wie Blut oder Blutbestandteilen ausgesetzt. Des weiteren wurde die Inaktivierungfähigkeit von VIPERIN1 an weiteren Viren nachgewiesen. Um eine möglichst hohe Heterogenität zu gewährleisten, wurden der Lamda-Bakteriophage als ebenfalls nicht umhülltes Virus jedoch mit dsDNA mit VIPERIN1 sowohl in Tris-Puffer pH 7.5 als auch in Blutplasma inkubiert sowie der BVDV, ein Modellvirus für behüllte Viren mit ebenfalls einzelsträngiger RNA in Puffer. In Figur 5 sind die Ergebnisse tabellarisch dargestellt. Die Vireninaktivierung ist wiederum im 10-er Logarithmus angegeben, wobei log 6 die Nachweisgrenze der Tests darstellte. Der sterile Tris-Puffer ist jeweils mit 6g Tris, 2g MgSO₄ *7H₂O, 5.8g NaCl auf 11 Wasser, sowie Zugabe 5ml 20%ige Albuminlösung angesetzt worden.

Es wurden jeweils 50µg/ml VIPERIN beladen mit Eisen(III) 4 Stunden bei Raumtemperatur mit je 1x10⁹ pfu Phagentiter pro ml Wasser und 5mM Natriumascorbat. Der MS2-Phage wurde dabei um mindestens 6 Logarithmusstufen in Puffer, Plasma, Erythrozytenkonzentrat und in Vollblut reduziert, in Thrombozytenkonzentrat um mehr als 5,6 Stufen. Der Lamda-Phage wurde in Puffer um mehr als 5,6 und in Plasma um mehr als 6 Logarithmusstufen reduziert, der BVDV in Puffer um mehr als 6.

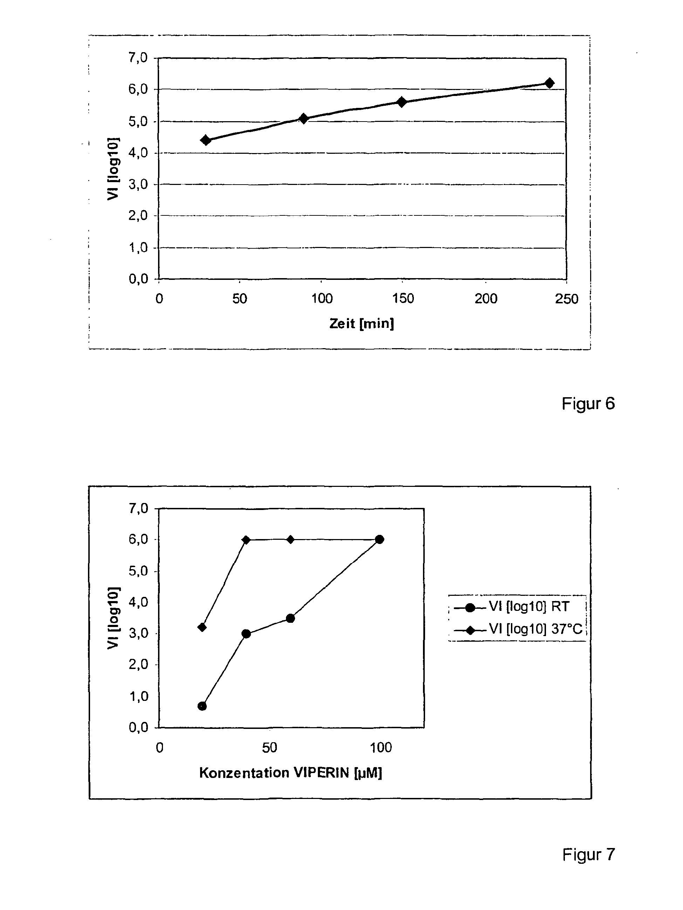

Darüber hinaus wurde die Wirksamkeit von VIPERIN beispielhaft durch die Inaktivierung des Phagen MS2 in Abhängigkeit von der Zeit, von der Temperatur bzw. von der Konzentration untersucht. Der Inkubationsansatz bestand jeweils aus einer Virusbeladung von 10⁹ pfu/ml, VIPERIN beladen mit Eisen(III) und 5mM Natriumascorbat. In Figur 6 ist graphisch das Ergebnis dargestellt, daß die Inaktivierung von MS2 über eine Zeitdauer von etwa 4 Stunden um fast 2 Logarithmusstufen ansteigt, die Inaktivierung also zeitabhängig ist.

Figur 7 zeigt das Ergebnis der Vireninaktivierung einmal bei Raumtemperatur (RT) und bei 37°C bei 4 Stunden Inkubation ebenfalls graphisch. Zum einen ist die Vireninaktivierung temperaturabhängig. Es wurde gefunden, daß bei geringeren VIPERIN-Konzentrationen die Inaktivierung bei 37°C höher ist, allerdings bereits bei etwa 40µM VIPERIN seinen maximalen Wert von mindestens 6 Logarithmusstufen Reduzierung der Virenaktivität zeigt. Zum anderen ist die Vireninaktivierung konzentrationsabhängig. Bei Inkubation bei Raumtemperatur steigt die Inaktivierung des MS2-Phagen mit zunehmender Konzentration an VIPERIN stetig an. Obgleich bei Inkubation bei 37°C der maximal zu messende Wert der Inaktivierung des MS2 von 6 Logarithmusstufen bereits bei 40µM VIPERIN erreicht ist, kann auch eine Steigerung der Inaktivierungsfähigkeit von 3 Logarithmusstufen ab 20µM VIPERIN detektiert werden.

## Patentansprüche

1. Pathogeninaktivierendes Mittel aus zwei funktionell getrennten Bereichen, mit einem an Nukleinsäure bindenden Element als ersten Bereich und einem die Nukleinsäure zerstörenden Konjugat als zweiten Bereich, sowie gegebenenfalls einem Spacer zwischen den beiden Bereichen,
wobei das an Nukleinsäure bindende Element aus der Gruppe der Olegonukleotide, der Interkalatoren, der Phosphatgruppengerüst-Binder und/oder der groove binders ausgewählt ist,
und das die Nukleinsäure zerstörende Konjugat ein Metallchelatkomplex ist, in welchem das Metall in verschiedenen Oxidationsstufen oxidierbar bzw. reduzierbar ist.

2. Pathogeninaktivierendes Mittel nach Anspruch 1, **dadurch gekennzeichnet, dass** der Metallchelatkomplex aus der Gruppe der inneren Komplexe oder der Gruppe der Komplexone ausgewählt ist.

3. Pathogeninaktivierendes Mittel nach Anspruch 1 und 2, **dadurch gekennzeichnet, dass** der Komplexbildner ausgewählt ist aus der Gruppe der Dioxime, Oxinate, EDTA-Komplexe, DTPA-Komplexe, EDTA-Komplexderivate, DTPA-Komplexderivate, Nitrilotriessigsäure und/oder Porphine hergestellt ist.

4. Pathogeninaktivierendes Mittel nach Anspruch 1 und 2, **dadurch gekennzeichnet, dass** das Metall des Metallchelatkomplexes aus der 8. Nebengruppe des Periodensystems ausgewählt ist.

5. Pathogeninaktivierendes Mittel nach Anspruch 1 und 2, **dadurch gekennzeichnet, dass** das Metall des Metallchelatkomplexes Eisen in der Oxidationsstufe II ist.

6. Pathogeninaktivierendes Mittel nach Anspruch 1 und 2, **dadurch gekennzeichnet, dass** das Metall des Metallchelatkomplexes Eisen in der Oxidationsstufe III ist.

7. Pathogeninaktivierendes Mittel nach einem oder mehreren der vorhergehenden Ansprüche, **dadurch gekennzeichnet, dass** der Metallchelatkomplex Eisen-(III)-EDTA ist.

8. Pathogeninaktivierendes Mittel nach einem oder mehreren der vorhergehenden Ansprüche, **dadurch gekennzeichnet, dass** zusätzlich ein Reduktionsmittel enthalten ist.

9. Pathogeninaktivierendes Mittel nach Anspruch 8, **dadurch gekennzeichnet, dass** das Reduktionsmittel Ascorbinsäure oder eines deren Salze ist.

10. Pathogeninaktivierendes Mittel nach einem oder mehreren der vorhergehenden Ansprüche, **dadurch gekennzeichnet, dass** die Ascorbinsäure oder deren Salz in einer Konzentration von mehr als 100µmol/l vorliegt.

11. Pathogeninaktivierendes Mittel nach Anspruch 1, **dadurch gekennzeichnet, dass** das Oligonukleotid einzelsträngig ist und weniger als 7 spezifische Nukleotide aus der Gruppe Thymin, Adenin, Cytosin, Guanin und/oder Uracil aufweist.

12. Pathogeninaktivierendes Mittel nach Anspruch 11, **dadurch gekennzeichnet, dass** das Oligonukleotid weitere universelle Basen ausgewählt aus der Gruppe Inosin und/oder 5-Nitroindol aufweist.

13. Pathogeninaktivierendes Mittel nach Anspruch 1, **dadurch gekennzeichnet, dass** die "groove binders" ausgewählt sind aus der Gruppe der "minor groove binder", enthaltend Distamycin, Mitomycin, Netropsin, Lexitropsine, Berenile, Indole und/oder Triarylmethan-Farbstoffe, und der Gruppe der "major groove binder" enthaltend Aflatoxine ausgewählt sind.

14. Pathogeninaktivierendes Mittel nach Anspruch 1, **dadurch gekennzeichnet, dass** Phosphatgruppengerüst-Binder ausgewählt sind aus der Gruppe der Spermine, Spermidine und anderen Polyaminen.

15. Pathogeninaktivierendes Mittel nach Anspruch 1, **dadurch gekennzeichnet, dass** die Interkalatoren ausgewählt sind aus der Gruppe der benzoiden Aromate und/oder der nicht benzoiden Aromate, wie Heteroaromate.

16. Pathogeninaktivierendes Mittel nach Anspruch 15, **dadurch gekennzeichnet, dass** die Aromate aus Mehrringsystemen bestehen.

17. Pathogeninaktivierendes Mittel nach Anspruch 15 und 16, **dadurch gekennzeichnet, dass** Aromate Acridine, Acridone, Alloxazine bzw. Isoaloxazine wie Riboflavin und andere Flavine, Porphine bzw. Porphyrine wie Häm, Mycine, Fluorene, Acridine, Psoralene, Ethidiumsalze, Oxirane, Coumarine, Psoralene, Phenylverbindungen, Xanthene, Ellipticene, Quinolone, Chloroquine, Quinine, Propidium Coralyne und/oder deren Derivate sind.

18. Pathogeninaktivierendes Mittel nach einem oder mehreren der vorhergehenden Ansprüche, **dadurch gekennzeichnet, dass** als Interkalator Riboflavin bevorzugt ist.

19. Pathogeninaktivierendes Mittel nach einem oder mehreren der vorhergehenden Ansprüche, **dadurch gekennzeichnet, dass** das pathogeninaktivierende Mittel Riboflavin mit kovalent gebundenem Eisen-EDTA-Komplex enthält, wobei der Komplex über einen Spacer an das Riboflavin gebunden sein kann.

20. Pathogeninaktivierendes Mittel nach Anspruch 1, **dadurch gekennzeichnet, dass** die Pathogene Nukleotid tragende Viren, Bakterien, Protozoen und/oder Pilze sind.

21. Verwendung eines pathogeninaktivierenden Mittels gemäß den Ansprüchen 1 bis 20 zur Inaktivierung von Pathogenen in Blutprodukten, **dadurch gekennzeichnet, dass** die Inaktivierung durch Anlagerung an Nukleinsäuren der Pathogene erfolgt und Zerstörung der Nukleinsäuren an dieser Stelle.

22. Verwendung nach Anspruch 21, **dadurch gekennzeichnet, dass** die Inaktivierung durch Zugabe eines Stoffes vorzugsweise eines Reduktionsmittels gestartet wird.

23. Verwendung nach Anspruch 22, **dadurch gekennzeichnet, dass** das Reduktionsmittel Ascorbinsäure oder eines deren Salze ist.

24. Verwendung nach Anspruch 21 bis 23, **dadurch gekennzeichnet, dass** die Inaktivierung in Flüssigkeiten erfolgt.

25. Verwendung nach Anspruch 21 bis 24, **dadurch gekennzeichnet, dass** die Flüssigkeiten physiologische Lösungen sind.

26. Verwendung nach Anspruch 25, **dadurch gekennzeichnet, dass** die physiologischen Lösungen Blut oder Blutbestandteile sind.

27. Verwendung gemäß den Ansprüchen 21 bis 26, **dadurch gekennzeichnet, dass** das Mittel zur Inaktivierung von Pathogenen oder dessen Bestandteile nach erfolgter Inaktivierung entfernt wird.

28. Verwendung gemäß den Ansprüchen 21 bis 26, **dadurch gekennzeichnet, dass** das Mittel zum Starten der Reaktion nach erfolgter Inaktivierung entfernt wird.

## Claims

1. A pathogen-inactivating agent comprising two functionally separate regions, with an element binding to nucleic acid as the first region and a conjugate destroying the nucleic acid as the second region, and optionally a spacer between the two regions,
wherein the element binding to nucleic acid is selected from the group of oligonucleotides, intercalators, the phosphate group framework binders and/or the groove binders,
and the conjugate destroying the nucleic acid is a metal chelate complex in which the metal can be reduced or oxidised at various oxidation levels.

2. A pathogen-inactivating agent according to claim 1 **characterised in that** the metal chelate complex is selected from the group of the internal complexes or from the group of the complexones.

3. A pathogen-inactivating agent according to claim 1 and claim 2 **characterised in that** the chelating agent is selected from the group of dioximes, oxinates, EDTA complexes, DTPA complexes, EDTA complex derivatives, DTPA complex derivatives, nitrilotriacetic acid and/or porphines.

4. A pathogen-inactivating agent according to claim 1 and claim 2 **characterised in that** the metal of the metal chelate complex is selected from the 8th secondary group of the periodic table.

5. A pathogen-inactivating agent according to claim 1 and claim 2 **characterised in that** the metal of the metal chelate complex is iron at the oxidation level II.

6. A pathogen-inactivating agent according to claim 1 and claim 2 **characterised in that** the metal of the metal chelate complex is iron at the oxidation level III.

7. A pathogen-inactivating agent according to one or more of the preceding claims **characterised in that** the metal chelate complex is iron-(III)-EDTA.

8. A pathogen-inactivating agent according to one or more of the preceding claims **characterised in that** a reduction agent is additionally included.

9. A pathogen-inactivating agent according to claim 8 **characterised in that** the reduction agent is ascorbic acid or one of the salts thereof.

10. A pathogen-inactivating agent according to one or more of the preceding claims **characterised in that** the ascorbic acid or salt thereof is present in a concentration of more than 100 µmol/l.

11. A pathogen-inactivating agent according to claim 1 **characterised in that** the oligonucleotide is single-stranded and has fewer than 7 specific nucleotides from the group thymine, adenine, cytosine, guanine and/or uracil.

12. A pathogen-inactivating agent according to claim 11 **characterised in that** the oligonucleotide has universal bases selected from the group of inosine and/or 5-nitroindol.

13. A pathogen-inactivating agent according to claim 1 **characterised in that** the groove binders are selected from the group of minor groove binders containing distamycin, mitomycin, netropsin, lexitropsins, berenils, indols and/or triarylmethane dyes, and the group of major groove binders' containing aflatoxins.

14. A pathogen-inactivating agent according to claim 1 **characterised in that** phosphate group framework binders are selected from the group of spermines, spermidines and other polyamines.

15. A pathogen-inactivating agent according to claim 1 **characterised in that** the intercalators are selected from the group of benzenoid aromatic compounds and/or non-benzenoid aromatic compounds such as heteroaromatic compounds.

16. A pathogen-inactivating agent according to claim 15 **characterised in that** the aromatic compounds comprise multi-ring systems.

17. A pathogen-inactivating agent according to claim 15 and claim 16 **characterised in that** aromatic compounds are acridine, acridone, alloxazine, or isoaloxazine such as riboflavin and other flavins, porphines or porphyrines such as heme, mycine, fluorene, acridine, psoralene, ethidium salts, oxirane, coumarine, psoralene, phenyl compounds, xanthene, ellipticene, quinolone, chloroquine, quinine, propidium coralyne and/or derivatives thereof.

18. A pathogen-inactivating agent according to one or more of the preceding claims **characterised in that** riboflavin is preferred as the intercalator.

19. A pathogen-inactivating agent according to one or more of the preceding claims **characterised in that** the pathogen-inactivating agent contains riboflavin with covalently bound iron-EDTA-complex, wherein the complex can be bound to the riboflavin by way of a spacer.

20. A pathogen-inactivating agent according to claim 1 **characterised in that** the pathogens are nucleotide-bearing viruses, bacteria, protozoa and/or fungi.

21. Use of a pathogen-inactivating agent according to claims 1 to 20 for the inactivation of pathogens in blood products, **characterised in that** inactivation is effected by attachment to nucleic acids of the pathogens and destruction of the nucleic acids at that location.

22. Use according to claim 21 **characterised in that** inactivation is started by the addition of a substance, preferably a reduction agent.

23. Use according to claim 22 **characterised in that** the reduction agent is ascorbic acid or one of the salts thereof.

24. Use according to claims 21 to 23 **characterised in that** inactivation is effected in liquids.

25. Use according to claims 21 to 24 **characterised in that** the liquids are physiological solutions.

26. Use according to claim 25 **characterised in that** the physiological solutions are blood or blood constituents.

27. Use according to claims 21 to 26 **characterised in that** the agent for the inactivation of pathogens or constituents thereof is removed after inactivation has been effected.

28. Use according to claims 21 to 26 **characterised in that** the agent for starting the reaction is removed after inactivation has been effected.

## Revendications

1. Agent inactivant des agents pathogènes en deux zones fonctionnellement séparées, avec un élément se liant sur l'acide nucléique comme première zone et un conjugué détruisant l'acide nucléique comme deuxième zone, ainsi que le cas échéant avec un espaceur entre les deux zones,
sachant que l'élément se liant sur l'acide nucléique est choisi parmi le groupe des oligonucléotides, des intercalateurs, des éléments se liant au squelette du groupe phosphate et/ou des groove binders,
et que le conjugué détruisant l'acide nucléique est un complexe de métal chélaté dans lequel le métal est oxydable, respectivement réductible, à différents degrés d'oxydation.

2. Agent inactivant des agents pathogènes selon la revendication 1, **caractérisé en ce que** le complexe de métal chélaté est choisi parmi le groupe des complexes internes ou parmi le groupe des Komplexons.

3. Agent inactivant des agents pathogènes selon les revendications 1 et 2, **caractérisé en ce que** l'agent complexant est choisi parmi le groupe des dioximes, des oxinates, des complexes de l'EDTA, des complexes du DTPA, des dérivés de complexes de l'EDTA, des dérivés de complexes du DPTA, de l'acide nitrilotriacétique et/ou des porphines.

4. Agent inactivant des agents pathogènes selon les revendications 1 et 2, **caractérisé en ce que** le métal du complexe de métal chélaté est choisi dans le 8^{e} groupe secondaire du système périodique.

5. Agent inactivant des agents pathogènes selon les revendications 1 et 2, **caractérisé en ce que** le métal du complexe de métal chélaté est du fer au degré d'oxydation II.

6. Agent inactivant des agents pathogènes selon les revendications 1 et 2, **caractérisé en ce que** le métal du complexe de métal chélaté est du fer au degré d'oxydation III.

7. Agent inactivant des agents pathogènes selon l'une ou plusieurs des revendications précédentes, **caractérisé en ce que** le complexe de métal chélaté est un complexe de fer (III) et d'EDTA.

8. Agent inactivant des agents pathogènes selon l'une ou plusieurs des revendications précédentes, **caractérisé en ce qu'**un agent réducteur est contenu en plus.

9. Agent inactivant des agents pathogènes selon la revendication 8, **caractérisé en ce que** l'agent réducteur est de l'aide ascorbique ou un de ses sels.

10. Agent inactivant des agents pathogènes selon l'une ou plusieurs des revendications précédentes, **caractérisé en ce que** l'acide ascorbique ou son sel sont présents en une concentration de plus de 100 µmol/l.

11. Agent inactivant des agents pathogènes selon la revendication 1, **caractérisé en ce que** l'oligonucléotide est à un brin et comprend moins de 7 nucléotides spécifiques parmi le groupe de la thymine, de l'adénine, de la cytosine, de la guanine et/ou de l'uracile.

12. Agent inactivant des agents pathogènes selon la revendication 11, **caractérisé en ce que** l'oligonucléotide comporte d'autres bases universelles parmi le groupe de l'inosine et/ou du 5-nitroindol.

13. Agent inactivant des agents pathogènes selon la revendication 1, **caractérisé en ce que** les « groove binders » sont choisis parmi le groupe des « minor groove binders », contenant la distamycine, la mitomycine, la nétropsine, les lexitropsines, les béréniles, les indoles et/ou les colorants au triarylméthane, et parmi le groupe des « major groove binders », contenant les aflatoxines.

14. Agent inactivant des agents pathogènes selon la revendication 1, **caractérisé en ce que** l'agent s'attachant aux groupes phosphates est choisi parmi le groupe des spermines, des spermidines et d'autres polyamines.

15. Agent inactivant des agents pathogènes selon la revendication 1, **caractérisé en ce que** les intercalateurs sont choisis parmi le groupe des aromatiques benzoïdes et/ou des aromatiques non benzoïdes, comme les hétéroaromatiques.

16. Agent inactivant des agents pathogènes selon la revendication 15, **caractérisé en ce que** les aromatiques consistent en systèmes polycycliques.

17. Agent inactivant des agents pathogènes selon les revendications 15 et 16, **caractérisé en ce que** les aromatiques sont des acridines, des acridones, des alloxazines, respectivement des isoaloxazines comme la riboflavine, les porphines, respectivement les porphyrines comme l'hème, les mycines, les fluorènes, les acridines, les psoralènes, les sels d'éthidium, les oxirannes, les coumarines, les psoralènes, les composés phénylés, les xanthènes, les ellipticènes, les quinolones, les chloroquines, les propidium coralynes et/ou leurs dérivés.

18. Agent inactivant des agents pathogènes selon l'une ou plusieurs des revendications précédentes, **caractérisé en ce que**, comme intercalateur, la riboflavine est préférée.

19. Agent inactivant des agents pathogènes selon l'une ou plusieurs des revendications précédentes, **caractérisé en ce que** l'agent inactivant des agents pathogènes contient de la riboflavine avec un complexe du fer et de l'EDTA lié par covalence, sachant que le complexe doit être lié à la riboflavine par un espaceur.

20. Agent inactivant des agents pathogènes selon la revendication 1, **caractérisé en ce que** les pathogènes sont des virus, des bactéries, des protozoaires et/ou des champignons porteurs de nucléotides.

21. Utilisation d'un agent inactivant des agents pathogènes selon les revendications 1 à 20 pour l'inactivation de pathogènes dans des produits obtenus à partir de sang, **caractérisée en ce que** l'inactivation s'effectue par liaison sur les acides nucléiques des agents pathogènes et par destruction des acides nucléiques en cet emplacement.

22. Utilisation selon la revendication 21, **caractérisée en ce que** l'inactivation est déclenchée par l'addition d'une substance, de préférence d'un agent réducteur.

23. Utilisation selon la revendication 22, **caractérisée en ce que** l'agent réducteur est de l'acide ascorbique ou un de ses sels.

24. Utilisation selon les revendications 21 à 23, **caractérisée en ce que** l'inactivation s'effectue dans des liquides.

25. Utilisation selon les revendications 21 à 24, **caractérisée en ce que** les liquides sont des solutions physiologiques.

26. Utilisation selon la revendication 25, **caractérisée en ce que** les solutions physiologiques sont du sang ou des composants du sang.

27. Utilisation selon les revendications 21 à 26, **caractérisée en ce que** l'agent d'inactivation des pathogènes ou ses composants sont retirés après que l'inactivation a été effectuée.

28. Utilisation selon les revendications 21 à 26, **caractérisée en ce que** l'agent pour le déclenchement de la réaction est retiré après que l'inactivation a été effectuée.
